# EUROPÄISCHE PATENTANMELDUNG

(11) **EP 2 039 296 A2**
(43) Veröffentlichungstag der Anmeldung: **25.03.2009**
(21) Anmeldenummer: 08105159.1
(22) Anmeldetag: 27.08.2008
(51) Int. Cl.: A61B 6/00, A61B 5/055, F21S 8/00

(54) **Beleuchtungsvorrichtung für eine medizinische Untersuchungsvorrichtung**

(30) Priorität: 21.09.2007 DE 102007045325
(71) Anmelder: Siemens Aktiengesellschaft, 80333 München (DE)
(72) Erfinder: Helmreich, Gerhard, 91090 Effeltrich (DE); Ramsauer, Martin, 90602 Pyrbaum (DE); Richenberger, Michael, 24340 Eckernförde (DE)

(57) **Zusammenfassung**

Medizinische Untersuchungsvorrichtung umfassend einen Untersuchungsbereich, in dem eine Untersuchungsperson unter Verwendung einer bildgebenden Einrichtung untersucht wird, wobei wenigstens eine flächige, über ihre Lichtabstrahlfläche (15) homogenes Licht emittierende Leuchteinrichtung (11) umfassend wenigstens ein Leuchtmittel (25, 25a, 25b) vorgesehen ist, mittels der zumindest ein Teil des Untersuchungsbereichs (16) und/oder des Bereichsumfelds (17) ausleuchtbar ist.

## Beschreibung

Die Erfindung betrifft eine medizinische Untersuchungsvorrichtung umfassend einen Untersuchungsbereich, in dem eine Untersuchungsperson unter Verwendung einer bildgebenden Einrichtung untersucht wird.

Derartige Untersuchungsvorrichtungen sind in unterschiedlichen Ausgestaltungen bekannt und dienen unter Verwendung verschiedener, auf jeweils unterschiedlichen Bildaufnahmetechniken basierender bildgebender Einrichtungen dazu, Bilder einer zu untersuchenden Person oder eines bestimmten Bereichs einer Person zu erstellen, die diagnostisch aussagefähig sind. Zu nennen sind beispielsweise Röntgenvorrichtungen umfassend eine Strahlungsquelle und einen Strahlungsempfänger, mittels denen Röntgenbilder aufgenommen werden können, wobei unterschiedliche Typen von Röntgenvorrichtungen, die mitunter auch bestimmten diagnostischen Fragestellungen dienen, bekannt sind. So beispielsweise ein Mammographiegerät, das zur Durchführung von Brustuntersuchungen dient, Röntgenvorrichtungen mit wandseitig angeordneten Detektoren für Aufnahmen einer stehenden Person, C-Bogen-Röntgenvorrichtungen, die häufig an Stativen und deckenseitigen Verfahrenwagen geführt sind, etc. Alternativ zu klassischen Röntgenvorrichtungen sind Computertomographiegeräte bekannt, die ebenfalls mit Röntgenstrahlung arbeiten, jedoch einen üblicherweise zylindrischen Untersuchungsbereich in Form einer Patientenbohrung aufweisen, in die der auf einem Patientenlagerungstisch befindliche Patient eingefahren wird. Um die Bohrung herum kreist eine Röntgenröhre, so dass Durchleuchtungsbilder aus beliebigen Positionen aufgenommen werden können. Bekannt sind ferner Magnetresonanzgeräte, die sich zur Bildaufnahme der Wechselwirkung zwischen einem angelegten Magnetfeld und der Spins der Elektronen der Atomkerne bedienen. Auch hierunter sind Geräte bekannt, die eine im Wesentlichen zylindrische Patientenaufnahme aufweisen, in die der Patient auf einem Tisch liegend eingefahren wird. Bekannt sind aber auch offene Systeme, die zwei vertikal übereinander angeordnete Magneten umfassen, zwischen die der Patient von der Seite her eingefahren werden kann. Die verschiedenen Typen medizinischer Untersuchungsvorrichtungen, deren Aufbau und Funktionsweise sowie deren Anwendungsgebiete sind dem Fachmann hinlänglich bekannt und bedürfen keiner detaillierten Erläuterung.

In nahezu allen Fällen ist es erforderlich, die Untersuchungsperson im Untersuchungsbereich korrekt zu positionieren, um sicherzustellen, dass der zu untersuchende Körperbereich korrekt im Zentrum der bildgebenden Einrichtung positioniert ist. Häufig sind an der Untersuchungsperson selbst vor dem Einbringen in den Untersuchungsbereich oder an der bereits dort befindlichen Untersuchungsperson Handlungen vorzunehmen, beispielsweise Geräte anzuschließen oder sogar kleinere chirurgische Eingriffe wie beispielsweise eine Biopsie zu nehmen oder einen Katheter zu setzen etc. Dies setzt voraus, dass die verantwortliche Person, sei es ein medizinischtechnisches Hilfspersonal oder ein Arzt, den Bereich, in dem sich die Untersuchungsperson befindet und in dem er die Handlung vornehmen will, hinreichend gut einsehen kann. Nachdem medizinische Untersuchungsvorrichtungen häufig in fensterlosen oder abgedunkelten Räumen stehen, was dem Patientenkomfort üblicherweise dienlich ist, bedient man sich regelmäßig deckenseitig angeordneter Leuchten, üblicherweise einfache Punktlichtquellen, die den Raum insgesamt ausleuchten. Über diese Leuchten ist also ein Hintergrundlicht erzeugbar, das auch den Untersuchungsbereich oder das Bereichsumfeld um den Untersuchungsbereich ausleuchten soll. Hierüber wird jedoch häufig nur eine unzureichende Ausleuchtung erreicht, mitunter besteht das Problem, dass die Untersuchungsperson oder die die Untersuchungsperson behandelnde Person selbst Schatten wirft.

Der Erfindung liegt damit das Problem zugrunde, eine medizinische Untersuchungsvorrichtung anzugeben, bei der eine verbesserte Ausleuchtung des Untersuchungsbereichs und/oder des Bereichsumfelds möglich ist.

Zur Lösung dieses Problems ist bei einer medizinischen Untersuchungsvorrichtung der eingangs genannten Art erfindungsgemäß wenigstens eine flächige, über ihre Lichtabstrahlfläche homogenes Licht emittierende Leuchteinrichtung umfassend wenigstens ein Leuchtmittel vorgesehen, mittels der zumindest ein Teil des Untersuchungsbereichs und/oder des Bereichsumfelds ausleuchtbar ist.

Die erfindungsgemäße Untersuchungsvorrichtung zeichnet sich dadurch aus, dass an ihr selbst eine Leuchteinrichtung vorgesehen ist, mittels der gezielt der Untersuchungsbereich und/oder das Bereichsumfeld um den Untersuchungsbereich ausgeleuchtet werden kann. Dabei kommt erfindungsgemäß eine Flächenleuchteinrichtung zum Einsatz, die ihr Licht nicht wie übliche Leuchteinrichtungen punktuell abstrahlt, sondern über eine große Lichtabstrahlfläche verfügt, die entsprechend den gegebenen Möglichkeiten beliebig groß gewählt werden kann. Je nachdem, um welche Art Untersuchungsvorrichtung es sich handelt, kann die Lichtabstrahlfläche bis zu 1 m² oder mehr betragen, wobei an der erfindungsgemäßen Untersuchungsvorrichtung natürlich auch mehrere derartiger Flächenleuchteinrichtungen vorgesehen sein können, die an unterschiedlichen Positionen angeordnet sind und unterschiedliche Größe aufweisen. In jedem Fall sind sie derart positioniert, dass sie gezielt den Untersuchungsbereich bzw. des Bereichsumfelds beleuchten können, der bzw. das optimal und homogen ausgeleuchtet werden kann, nachdem die Leuchteinrichtung über eine große Lichtabstrahlfläche homogenes Licht emittiert.

Die großflächige, homogene Ausleuchtung der relevanten Bereiche der Untersuchungsvorrichtung bietet der die Untersuchung durchführenden Person die Möglichkeit, den homogen ausgeleuchteten Bereich optimal einsehen zu können, sei es, dass sie in diesem Bereich arbeitet, um beispielsweise ein Gerät (z.B. eine Spule im Falle eines Magnetresonanzgeräts, den Kompressionsplatte eines Mammographiegeräts etc.) anzubringen oder zu positionieren, sei es lediglich zur kontinuierlichen Kontrolle während der Untersuchung, beispielsweise zur Erfassung etwaiger Patientenbewegungen oder dergleichen. Die homogene Lichtabstrahlung stellt sicher, dass nicht nur eine punktuelle Ausleuchtung gegeben ist, sondern dass ein großes Volumen homogen ausgeleuchtet wird. Insgesamt wird hierüber das Arbeiten an und mit der erfindungsgemäßen Untersuchungsvorrichtung verbessert, etwaige Fehler, die möglicherweise auf eine schlechte Ausleuchtung zurückzuführen sind, können vermieden werden.

Ein weiterer beachtlicher Vorteil der erfindungsgemäßen Untersuchungsvorrichtung ist ferner, dass die flächige, homogene Beleuchtung eine angenehmere Atmosphäre bietet, als die bisher bekannte deckenseitige Raumbeleuchtung, insbesondere, wenn die erfindungsgemäße Flächenleuchteinrichtung auch während der Untersuchung in Betrieb ist. Das heißt, dass hierüber auch der Patientenkomfort verbessert werden kann, verbunden auch hier mit dem Effekt, dass selbstverständlich auch der Patient sich im ausgeleuchteten Untersuchungsbereich oder des Bereichsumfelds infolge der optimalen Beleuchtung desselben wesentlich besser orientieren kann und beispielsweise etwaigen Kommandos der die Untersuchung durchführenden Person, beispielsweise bezüglich der Positionierung, oder etwaigen Bewegungen eines Teils der Untersuchungsvorrichtung etc. wesentlich besser folgen kann.

Wie beschrieben kann die Flächengröße der Leuchteinrichtung bzw. deren Lichtabstrahlfläche letztlich beliebig gewählt werden, soweit die Leuchteinrichtung an oder in der Untersuchungsvorrichtung und dort am oder im Untersuchungsbereich oder dem Bereichsumfeld integriert werden kann. Die Leuchteinrichtung ist plattenförmig und je nach Anbringungsort eben oder gebogen ausgebildet. Sie begrenzt zweckmäßigerweise den Untersuchungsbereich oder das Bereichsumfeld zumindest teilweise, so dass sie den Untersuchungsbereich oder das Bereichsumfeld direkt ausleuchten kann. Die Leuchteinrichtung bildet also eine Art Verkleidungsteil, das beispielsweise auf die ohnehin vorgesehene Verkleidung der Untersuchungseinrichtung aufgesetzt bzw. an dieser befestigt wird, oder diese ersetzt. Hierüber ist eine einfache, wirkungsvolle und gleichzeitig optisch ansprechende Integration der Leuchteinrichtung selbst in bereits bekannte Untersuchungsvorrichtungen möglich.

Eine erfindungsgemäß verwendbare Flächenleuchteinrichtung weist ein lichtleitendes Flächenelement auf, das wiederum ein Mittel zur Umlenkung von seitlich über das wenigstens eine Leuchtmittel eingekoppelten Lichts zur Abstrahlfläche aufweist. Das lichttransparente Flächenelement ist beispielsweise aus Glas oder vorzugsweise aus Kunststoff, was die Formgebung erleichtert. Als Kunststoff ist beispielsweise PMMA oder PC verwendbar. Das Flächenelement weist, abhängig von seiner gesamten Größe, eine Dicke von wenigen Millimetern, z.B. 5 mm, bis zu wenigen Zentimetern, z.B. 2 cm, auf. Hierüber kann ihm eine hinreichende Eigensteifigkeit verliehen werden, auch wenn das Flächenelement selbst sehr groß ist und eine Fläche von einem oder mehr Quadratmetern belegt. Aus Platzgründen hat es sich als besonders zweckmäßig erwiesen, wenn das Leuchtmittel seitlich, also im Bereich einer Seitenkante, angeordnet ist, und von der Seite her in das lichttransparente, lichtleitende Flächenelement das emittierte Licht einkoppelt. Dieses wird im lichtleitenden Flächenelement primär parallel zur Lichtabstrahlfläche geführt. Über ein entsprechendes Umlenkmittel an dem Flächenelement wird sichergestellt, dass das eingekoppelte Licht reflektiert und zur Lichtabstrahlfläche hin umgelenkt wird, von der es dann austritt. Dieses Umlenkmittel ist so beschaffen, dass die Umlenkung zur Lichtabstrahlfläche hin im gesamten Bereich der Lichtabstrahlfläche erfolgt, bzw. - sofern nur über einen Teil der Lichtabstrahlfläche Licht emittiert werden soll - zumindest in diesem Bereich die Umlenkung erfolgt.

Eine Realisierungsmöglichkeit eines solchen Umlenkmittels ist beispielsweise in Form einer auf einer Seite des Flächenelements aufgebrachten strukturierenden Beschichtung zu sehen. Diese Beschichtung, die beispielsweise in einem Siebdruckverfahren auf das Glas- oder Kunststoffflächenelement aufgebracht wird, bildet eine Vielzahl an Reflexionszentren aus, weist also an der Grenzfläche zum Flächenelement eine Mikrostrukturierung auf, die die Reflexionszentren bildet. Alternativ zum Aufbringen einer solchen Beschichtung ist es auch denkbar, die Oberfläche des Flächenelements selbst zu strukturieren, also in der Oberfläche selbst die Reflexionszentren auszubilden, was beispielsweise durch mechanische, chemische oder physikalische Oberflächenbehandlung möglich ist. Das gezielte Aufbringen oder Ausbilden des Umlenkmittels, sei es über die Beschichtung oder die strukturierte Oberfläche selbst, ermöglicht es des Weiteren, den Bereich der Lichtabstrahlfläche, wo also tatsächlich Licht abgestrahlt werden soll, bzw. die Lichtabstrahlfläche selbst in beliebiger Form zu definieren, unabhängig von der eigentlichen Form des lichtleitenden Flächenelements. Wenn also beispielsweise das lichtleitende Flächenelement aufgrund entsprechender Montageerfordernisse größer auszuführen ist, als die eigentliche Lichtabstrahlfläche, so besteht hierüber die Möglichkeit, das Umlenkmittel auch nur in dem Bereich anzubringen oder auszubilden, wo tatsächlich Licht abgestrahlt werden soll. Auch ist selbstverständlich die Ausbildung entsprechender Abstrahlmuster möglich, indem das Umlenkmittel selbst eine bestimmte geometrische Flächenform belegt etc.

Um die Homogenität der Lichtabstrahlung noch weiter zu verbessern, weist das Flächenelement ferner an der Seite der Lichtabstrahlfläche einen Diffusor auf bzw. ist ihm ein solcher Diffusor zugeordnet. Ein solcher Diffusor ist zweckmäßigerweise ebenfalls als flächiges, plattenförmiges Element ausgebildet, das wie auch das lichtleitende Flächenelement eben oder gebogen ausgeführt ist. Denkbar ist hier beispielsweise die Verwendung einer sattinierten Glas- oder Kunststoffscheibe, die auf das Glas- oder Kunststoffflächenelement aufgesetzt wird. Auch dieses flächige Diffusorbauteil weist eine geringe Dicke von nur wenigen Millimetern auf, z.B. 2 - 10 mm. An der gegenüberliegenden Seite des Flächenelements ist zweckmäßigerweise eine Rückwand angeordnet, die die Leuchteinrichtung nach hinten hin abschießt.

Eine besonders zweckmäßige Weiterbildung der Erfindung sieht entlang der Seite mehrere Leuchtmittel zum Einkoppeln von Licht in das Flächenelement vor. Insbesondere bei relativ großflächigen Flächenelementen und mithin langen Seitenkanten und großer Lichtabstrahlfläche bietet sich so die Möglichkeit, eine Vielzahl kantenseitiger Lichterzeugungszentren und Lichteinkoppelpunkte zu realisieren. Neben der Möglichkeit, infolge der seitlichen Anordnung die Leuchteinheit insgesamt sehr schmal aufzubauen, bietet die Verwendung und Anordnung mehrerer seitlicher Leuchtmittel die Möglichkeit, auch eine große Lichtmenge in das Flächenelement einkoppeln zu können. Die Leuchtmittel selbst sind zweckmäßigerweise in einer bandartigen Einheit zusammengefasst, die entlang der Flächenelementseitenkante verlegt und dort entsprechend fixiert bzw. gekapselt angeordnet ist.

Wenngleich grundsätzlich Leuchtmittel in Form von Glüh- oder Halogenleuchtkörpern verwendet werden können, sieht eine zweckmäßige Erfindungsausgestaltung den Einsatz von LEDs vor. Diese sind sehr klein, können also ohne weiteres auch im Bereich schmaler Flächenelementseitenkanten angeordnet werden, bieten aber gleichzeitig eine sehr hohe Lichtausbeute. Ihr Betrieb ist energetisch günstig, die Lebensdauer ist lang, sie zeigen kaum Wärmeentwicklung. Auch ist es ohne weiteres möglich, auf sehr kleinem Raum viele LEDs integrieren zu können.

Wie bereits beschrieben, werden das oder die Leuchtmittel im montierten Zustand zweckmäßigerweise gekapselt, so dass sie sicher fixiert sind. Zweckmäßigerweise wird weiterhin an der Leuchteinrichtung im Bereich der Lichtabstrahlfläche ein das oder die Leuchtmittel verdeckendes nicht transparentes Abdeckelement angeordnet, das zumindest so groß ist, dass es von der Lichtabstrahlfläche her gesehen die Leuchtmittel überdeckt. Hierüber wird verhindert, dass eine Person von der Lichtabstrahlfläche her unmittelbar auf ein im Betrieb mitunter sehr helles Leuchtmittel blicken kann. Ein solches nicht transparentes Abdeckelement kann beispielsweise eine auch optisch ansprechende Metallplatte oder -schiene sein, die im Bereich der Seitenkante angeordnet ist bzw. umläuft. Auch Abdeckelemente aus Kunststoff sind selbstverständlich denkbar.

Die Leuchteinrichtung selbst umfasst des Weiteren eine Steuerungseinrichtung, über welche die Helligkeit des oder der Leuchtmittel, insbesondere der LEDs veränderbar ist. Die Helligkeit und damit die über die Lichtabstrahlfläche abgestrahlte Lichtmenge kann demgemäß über die Steuerungseinrichtung, die bei Verwendung mehrerer Leuchtmittel diese entweder gemeinsam oder auch separat ansteuern und mithin anschalten kann, je nach Bedarf und Situation anpassen bzw. verändern. Wird beispielsweise zum Positionieren des Patienten oder zum Anschließen oder Anbringen von Geräten oder zur Durchführung chirurgischer Eingriffe etc. eine hohe Helligkeit benötigt, so wird beispielsweise während der Untersuchung, also der Bildaufnahme, die mithin über mehrere Minuten bis Stunden gehen kann, eine reduzierte Helligkeit als ausreichend erachtet. Dies kann über die Steuerungseinrichtung ohne weiteres gesteuert werden, beispielsweise indem die Helligkeit über die Steuerungseinrichtung zwischen diskreten Helligkeitsstufen schaltbar ist, oder stufenlos zwischen einem Maximalwert und einem Minimalwert gedimmt werden kann. Hierüber ist also eine beliebige Veränderung der Helligkeit möglich, was auch die Möglichkeit bietet, bei Bedarf und insbesondere langer Untersuchungsdauer eine den Patientenkomfort hebende ansprechende Atmosphäre zu erzeugen.

Eine besonders zweckmäßige Erfindungsausgestaltung sieht ferner vor, mittels der Leuchteinrichtung weißes Licht und Licht wenigstens einer Farbe emittieren zu können. Das heißt, dass bei Bedarf von einer weißen Beleuchtung auf eine Farbbeleuchtung über die Steuerungseinrichtung umgeschaltet werden kann. Dies bietet die Möglichkeit, der Untersuchungsperson Informationen zu vermitteln, beispielsweise dahingehend, dass während der Bildaufnahme von weißer Beleuchtung auf Farbbeleuchtung umgeschaltet wird, so dass die Untersuchungsperson genau weiß, wann die Bildaufnahme beginnt. Am Ende derselben kann wieder von der Farbbeleuchtung auf die weiße Beleuchtung umgeschaltet werden, so dass hierüber das Ende der Bildaufnahme mitgeteilt werden kann. Denkbar ist es auch, hierüber Zeiträume anzuzeigen, innerhalb welcher der Patient sich beispielsweise nicht bewegen darf oder die Luft anhalten muss oder bestimmte Bewegungen durchführen muss, wenn dies eine bestimmte Bildaufnahme, beispielsweise im Bereich der Magnetresonanzbildgebung, erfordert. Wenn also der Patient beispielsweise die Luft anhalten soll, wird ihm dies durch Umschalten auf Farblicht angezeigt, das so lange zugeschaltet bleibt, bis der Luftanhaltezeitraum verstrichen ist und wieder auf Weißlicht umgeschaltet werden kann. Hierüber kann auf sehr einfache und intuitive Weise eine Information vermittelt werden. Denkbar ist beispielsweise ein Umschalten auf Rotlicht, da der Mensch mit rotem Licht häufig eine Informationsvermittlung verbindet.

Hierüber ist es aber auch ohne weiteres möglich, den Patientenkomfort zu verbessern und die Beleuchtungsatmosphäre zu beeinflussen, was insbesondere dann von Vorteil ist, wenn es sich um lang dauernde Untersuchungen handelt. Denn es besteht dann die Möglichkeit, unter Abschalten der hellen Weißlichtbeleuchtung auf eine angenehme Farbausleuchtung zu wechseln, die vom Patienten grundsätzlich als angenehm empfunden wird und so sein Wohlbefinden während der Untersuchungsdauer fördert. Auch hat farbiges Licht mitunter einen therapeutischen Effekt, mitunter unterstützt und fördert es etwaige Heilungsprozesse, so dass auch diesbezüglich die Vorteile einer Farbveränderbarkeit genutzt werden können. Natürlich besteht auch hier die Möglichkeit, je nach Ausgestaltung der Leuchtmittel, von weißem Licht auf Farblicht stufenlos zu wechseln, beispielsweise das Weißlicht herunterzudimmen und das Farblicht heraufzudimmen, gleichwie auch eine scharfe Umschaltung von Weiß- auf Farblicht möglich ist.

Grundsätzlich besteht die Möglichkeit, weißes und farbiges Licht jeweils über die gesamte Lichtabstrahlfläche zu emittieren, mithin also für die unterschiedlichen Farbausleuchtungen stets die gesamte Lichtabstrahlfläche zu nutzen. Alternativ ist es auch denkbar, weißes und farbiges Licht in voneinander getrennten Bereichen der Lichtabstrahlfläche abzustrahlen. Dies ist vor allem dann zweckmäßig, wenn hierüber Informationen vermittelt werden sollen. Denn in diesem Fall ist es möglich, an der Lichtabstrahlfläche in einem bestimmten Bereich über die Farblichtbeleuchtung quasi eine Art "Informationsleuchte" zu realisieren.

Eine erste Erfindungsalternative sieht vor, den jeweiligen Leuchtfarben dezidierte Leuchtmittel zuzuordnen. Die mehrere Leuchtmittel umfassende Leuchteinrichtung umfasst also neben wenigstens einem weißes Licht emittierenden Leuchtmittel wenigstens ein farbiges Licht emittierendes Leuchtmittel, das separat ansteuerbar ist.

Alternativ dazu besteht die Möglichkeit, weißes Licht sowie beliebige Mischfarben des abgestrahlten Lichts durch Lichtmischung zu erzeugen. Zu diesem Zweck kann wenigstens ein rotes Licht, wenigstens ein blaues Licht und wenigstens ein grünes Licht emittierendes Leuchtmittel vorgesehen sein, die separat ansteuerbar sind. Je nachdem, welche Leuchtmittel zugeschaltet sind und mit welcher Intensität sie betrieben werden, kann weißes Licht oder eine beliebige andere Mischfarbe erzeugt werden. Zweckmäßigerweise kommen in allen Fällen LEDs zum Einsatz, also sowohl weiße als auch farbige LEDs. Handelt es sich um eine hinreichend große Leuchteinrichtung bzw. ein hinreichend großes lichttransparentes Flächenelement, so kommen von jeder Farbe selbstverständlich eine Vielzahl einzelner Leuchtmittel bzw. LEDs zum Einsatz, wobei jeweils drei LEDs der unterschiedlichen Farben zu Gruppen zusammengefasst und benachbart angeordnet sind. Die abgestrahlte Lichtfarbe ist bei dieser Erfindungsalternative zweckmäßigerweise ebenfalls stufenlos veränderbar, um einen sanften Übergang zu jeder beliebigen Farbe zu gewährleisten.

Neben dem Umstand, auch hierüber Informationen vermitteln zu können, bietet diese Erfindungsausgestaltung die Möglichkeit, beliebige Farbausleuchtungen und damit Lichtatmosphären zu schaffen. Dies kann sogar so weit gehen, dass die Untersuchungsperson insbesondere bei länger dauernden Untersuchungen eine gewünschte Farbe wählen kann, die während der Untersuchung abgestrahlt werden soll, bei der sie sich also am wohlsten fühlt. Für die untersuchende Person besteht trotz Farbausleuchtungen nach wie vor die Möglichkeit, den Patienten zu beobachten, nachdem auch die Farbausleuchtung stets den Untersuchungsbereich und/oder dessen Bereichsumfeld erfasst. Ein kontinuierlicher Farbwechsel ist hiermit ebenfalls möglich, z. B. um den Patientenkomfort bei lang dauernden Untersuchungen zu fördern. Hierbei findet dauernd ein Farbwechsel statt, wobei stetig von einer Farbe auf eine andere gewechselt wird. Vom Patienten wird ein solches Lichtspiel als angenehm und beruhigend empfunden.

Wie bereits beschrieben besteht über die Emission von Farbe grundsätzlich die Möglichkeit, dem Untersuchungspatienten Informationen vermitteln zu können. Um die Informationstiefe noch weiter zu erhöhen, sieht eine zweckmäßige Erfindungsausgestaltung vor, an oder in der Leuchteinrichtung wenigstens ein der Darstellung von Informationen und/oder Bildern dienendes Display anzuordnen, oder der Leuchteinrichtung ein solches Display zuzuordnen. Grundsätzlich besteht die Möglichkeit, in einem ausgezeichneten Bereich der Leuchteinrichtung ein entsprechendes Display zu integrieren, wozu beispielsweise ein entsprechender Ausschnitt in dem Flächenelement und dem davor gesetzten Diffusor respektive auch der Rückwand vorgesehen wird, in der ein solches Display integriert wird. Hierüber können dem Patienten beliebige Informationen dargestellt werden, beispielsweise wie lange die Untersuchung bereits dauert oder noch dauern wird, etwaige Handlungsanweisungen wie beispielsweise die Luft anzuhalten etc., oder Informationen für die zur Untersuchung durchführende Person wie beispielsweise im Falle eines Mammographiegeräts über die auf die Brust ausgeübte Kompressionskraft, die Kompressionsdichte, den Aufnahmewinkel oder allgemeine Patientendaten. Alternativ oder zusätzlich zu einem solchen Display ist es auch möglich, wenigstens eine Projektionseinrichtung vorzusehen, mittels der Informationen und/oder Bilder auf einen Bereich der Lichtabstrahlfläche der Leuchteinrichtung projizierbar sind. Hier kommt also ein separates Projektionsgerät zum Einsatz, das entsprechende Informationen/Bilder auf die Leuchteinrichtung strahlt.

Über die Farblichterzeugung besteht nicht nur die Möglichkeit, Informationen zu vermitteln oder eine angenehme Untersuchungsatmosphäre zu schaffen, sondern auch die Möglichkeit, durch Farbwechsel visuelle Reize zu geben, wie sie beispielsweise im Rahmen einer funktionellen Bildgebung, insbesondere bei Magnetresonanz- oder Computertomographieaufnahmen, mitunter erforderlich sind. Wird von Weißlicht beispielsweise auf grelles Rotlicht umgeschalten, so werden bestimmte Aktivitäten im Gehirn angeregt, die über die funktionelle Bildgebung erkannt werden können. Über die Darstellung von beliebigen Informationen über ein Display oder mittels einer Projektionseinrichtung ist es ferner möglich, andere optische Reize, die andere Gehirnzentren anregen, beispielsweise beim Lesen oder dergleichen, gezielt zu geben.

Wie beschrieben kann es sich bei der Untersuchungseinrichtung um eine Röntgeneinrichtung handeln, beispielsweise eine solche, die einer Untersuchung einer stehenden Untersuchungsperson dient, wobei in diesem Fall die Leuchteinrichtung vertikal stehend angeordnet ist. Ein solches Gerät kann beispielsweise ein Mammographiegerät sein, wobei die Leuchteinrichtung hinter dem an einer Vertikalsäule vertikal bewegbar angeordneten Röntgenbildaufnahmemittel angeordnet ist. Bei einem Mammographiegerät sind bekanntlich die Röntgenröhre und der Detektor, üblicherweise ein digitaler Flachdetektor, in einem festen Abstand zueinander über eine geeignete Bewegungsmechanik an einer Vertikalsäule bewegbar. Vor dieser Vertikalsäule, diese quasi nach vorne hin verkleidend, ist nun erfindungsgemäß die Leuchteinrichtung angeordnet. Die Leuchteinrichtung, hier eine ebene Platte, schaut je nach Ausgestaltung zur Seite bzw. oberhalb des Röntgenbildaufnahmemittels hervor, so dass der vor der Leuchteinrichtung befindliche Raum, das Umfeld des Untersuchungsbereichs, wie auch der Untersuchungsbereich zwischen Röntgenröhre und Detektor hierüber teilweise oder vollständig ausgeleuchtet werden können. Die Patientin steht vor dem Röntgenbildaufnahmemittel und damit vor der Leuchteinrichtung, blickt also in Richtung der beleuchteten Leuchteinrichtung. Die die Untersuchung vornehmende Person kann von der Seite her an das Röntgenbildaufnahmemittel herantreten, kann also mithin den Untersuchungsbereich optimal einsehen, und die Patientin positionieren, wie auch die Kompressionseinheit, über die die Brust komprimiert wird, bedienen. Der bzw. die relevanten Bereiche sind über die flächige Leuchteinrichtung optimal ausgeleuchtet, wie der Patientin auch ein lichttechnisch angenehmes Umfeld geboten wird, insbesondere wenn im Falle der Untersuchung die Leuchthelligkeit zurückgenommen oder auf eine andere Farbe umgeschaltet wird.

Während bei einem Mammographiegerät Röhre und Detektor gemeinsam zur Aufnahme vertikaler Durchleuchtungsbilder vertikal bewegt werden können, sind bei anderen Röntgengeräten horizontale Bildaufnahmen möglich. Häufig ist der Detektor vertikal beweglich an einer Vertikalsäule geführt, um ihn in unterschiedliche Positionen relativ zum davor stehenden Patienten positionieren zu können. Die Röntgenröhre befindet sich an einem Boden- oder Deckenstativ und kann unabhängig vom Detektor bewegt werden. Mit solchen Röntgengeräten sind beispielsweise Thoraxaufnahmen oder dergleichen möglich. Auch bei solchen Geräten ist nun die Integration einer erfindungsgemäßen Flächenleuchteinrichtung möglich, auch hier ist sie hinter dem Detektor und vor der Vertikalsäule angeordnet, verkleidet mithin also wiederum die Vertikalsäule und strahlt unmittelbar in den Untersuchungsbereich bzw. das Bereichsumfeld nach vorne hin ab.

In allen Fällen, in denen ein Teil des Röntgenbildaufnahmemittels oder das gesamte Röntgenbildaufnahmemittel vertikal bewegbar an einer Säule geführt ist, bietet sich die Anordnung der Leuchteinrichtung zwischen Säule und beweglichem Bildaufnahmeteil an. Die Leuchteinrichtung weist hierfür zweckmäßigerweise eine U-Form auf, wobei ein das Röntgenbildaufnahmemittel oder den Detektor tragender und an der Vertikalsäule geführter Tragarm zwischen den beiden U-Schenkeln durchgreift. Die Leuchteinrichtung ist hier also ein einteiliges Bauteil, das quasi nach Art eines Rahmens die Röntgeneinrichtung umläuft und von dem Tragarm, der die Verbindung zwischen Bildaufnahmemittel/Detektor und Vertikalsäule bietet, durchgriffen ist. Hierüber wird sichergestellt, dass eine sehr große Lichtabstrahlfläche gegeben ist, bei gleichzeitig vorteilhafter Integration der Leuchteinrichtung, die darüber hinaus auch eine Nachrüstmöglichkeit für bestehende Gerätschaften bietet.

Ein weiterer Typ von Röntgeneinrichtungen zeichnet sich dadurch aus, dass das Röntgenbildaufnahmemittel an einem Stativ, das an einem an deckenseitig angeordneten Führungsmitteln bewegbar geführten Wagen angeordnet ist, angeordnet ist, und ein Lagerungstisch für das Untersuchungsobjekt vorgesehen ist, wobei die Leuchteinrichtung an dem Wagen angeordnet ist. Hierbei handelt es sich um ein deckengeführtes Gerät, beispielsweise mit einem C-Bogen, an dem die Röntgenröhre und der Detektor angeordnet sind, oder zwei am Führungswagen angeordneten separaten Stativen, von denen eines die Röhre und das andere den Detektor trägt. Bei dieser Erfindungsausgestaltung ist die Leuchteinrichtung am Wagen angeordnet, der in der Regel relativ großflächig ist, nachdem er eine x-y-Führung an den deckenseitigen Führungsmitteln ermöglicht. Die Form der Leuchteinrichtung ist abhängig von der Wagenform, kann also beispielsweise viereckig oder rechteckig sein, denkbar auch rund, je nachdem, wie der Wagen ausgestaltet ist. Auch hier ist die Verwendung einer großflächigen Leuchteinrichtung möglich, die von oben direkt auf den Untersuchungsbereich, hier den Lagerungstisch mit dem Patienten, strahlt.

Ein anderer Typ einer Untersuchungseinrichtung ist wie beschrieben eine Magnetresonanzeinrichtung oder eine Computertomographieeinrichtung. Beide weisen zumeist eine im Wesentlichen zylindrische Aufnahmebohrung für die Untersuchungsperson auf, wobei die Leuchteinrichtung diese zumindest über einen Teil ihrer Länge und zumindest über einen Teil ihres Umfangs auskleidet. Hier ist die Leuchteinrichtung in das Innere der Aufnahmebohrung integriert, vornehmlich im Bereich des Randes der Aufnahmebohrung und bevorzugt an der Seite, an der der Patientenlagerungstisch eingefahren wird. In diesem Bereich ist die Integration der Leuchteinrichtung unkritisch, als die bildgebenden Magnetfelder mehr im Bereich der Bohrungsmitte erzeugt werden. Gleichwohl kann die gesamte Aufnahmebohrung als Untersuchungsbereich angesprochen werden bzw. der Bohrungseingang als Untersuchungsbereichsumfeld, in dem unter Verwendung der Leuchteinrichtung die erfindungsgemäße homogene Flächenausleuchtung möglich ist. Die Leuchteinrichtung ist hier infolge der Zylinderform der Aufnahmebohrung gebogen, sie kann sich beispielsweise über eine Länge von 30 oder 40 cm in das Bohrungsinnere erstrecken und beispielsweise um 180° bei Anordnung an der oberen Zylinderwand in der Mitte erstrecken, auch ein weiterer Umfangsumlauf ist möglich. Üblicherweise befindet sich in diesem Bereich der Kopf des Patienten, der dann beispielsweise Farbinformationen oder dergleichen vermittelt bekommen kann, verbunden mit der optimalen Ausleuchtung dieses Bereichs für die die Untersuchung durchführende Person. Auch kann eine entsprechende Farblichtatmosphäre oder Helligkeitsveränderung erzeugt bzw. vorgenommen werden, je nach Ausgestaltung der Leuchteinrichtung und Bedarf.

Alternativ oder zusätzlich zur Anordnung der Leuchteinrichtung in der Aufnahmebohrung ist es auch denkbar, bei einem Gerät mit einer solchen zylindrischen Aufnahmebohrung die Leuchteinrichtung an der Geräteaußenseite, hier der Stirnseite, von welcher Seite her der Patientenlagerungstisch mit dem Patienten in der Aufnahmebohrung eingefahren wird, anzuordnen, wobei die Leuchteinrichtung zumindest einen Teil der im Wesentlichen ringförmigen Stirnseite benachbart zur Aufnahmebohrung belegt. Über diese Leuchteinrichtung wird das Umfeld vor dem Aufnahmebereich, hier also vor der Untersuchungseinrichtung selbst, ausgeleuchtet. Die Leuchteinrichtung kann beispielsweise als Halbring ausgeführt sein und nur um die obere Hälfte laufen, oder sich weiter herum erstrecken. Über diese Leuchteinrichtung kann der Bereich vor der Bohrung mit ausgefahrenem Tisch sehr gut ausgeleuchtet werden, so dass die die Untersuchung durchführende Person beim Aufbringen des Patienten auf den Tisch und Setzen etwaiger Lokalspulen etc. dies in einem optimalen homogen ausgeleuchteten Umfeld vornehmen kann.

Eine Alternativausführung einer Magnetresonanzeinrichtung sieht anstelle einer zylindrischen Aufnahmebohrung mit zylindrischem Magneten zwei horizontal übereinander angeordnete und voneinander beabstandete Magneten vor, zwischen denen sich der Untersuchungsbereich befindet, wobei die Leuchteinrichtung am oberen Magneten, diesen zumindest teilweise belegend, angeordnet ist. Bei diesem offenen System kann der Patient von vorne oder der Seite her zwischen die Magneten eingefahren werden, sollte eine Handlung am Patienten ausgeführt werden, so ist er zwischen den beiden Magneten zugänglich. Die Leuchteinrichtung ist hier nun am oberen Magneten bzw. dessen Verkleidung angeordnet, strahlt also nach unten. Beispielsweise kann sie als umlaufender Ring oder Halbring am Randbereich des Magneten angeordnet sein.

Weitere Vorteile, Merkmale und Einzelheiten der Erfindung ergeben sich aus den im folgenden beschriebenen Ausführungsbeispielen sowie anhand der Zeichnungen. Dabei zeigen:
- Fig. 1: eine Prinzipdarstellung einer erfindungsgemäßen Untersuchungseinrichtung in Form eines Mammographiegeräts mit Leuchteinrichtung,
- Fig. 2: das Mammographiegerät aus Fig. 1 ohne Leuchteinrichtung,
- Fig. 3: eine Frontansicht der Leuchteinrichtung aus Fig. 1,
- Fig. 4: eine Teilansicht im Schnitt in Richtung der Linie IV - IV in Fig. 3,
- Fig. 5: eine vergrößerte Teilansicht des Bereichs V in Fig. 3,
- Fig. 6: eine Teilansicht im Schnitt in Richtung der Linie VI - VI in Fig. 3,
- Fig. 7: eine Prinzipdarstellung eines weiteren Typs einer erfindungsgemäßen Untersuchungseinrichtung in Form einer C-Bogen-Röntgeneinrichtung,
- Fig. 8: eine Prinzipdarstellung einer erfindungsgemäßen Untersuchungseinrichtung in Form einer Magnetresonanz- oder Computertomographieeinrichtung, und
- Fig. 9: eine Prinzipdarstellung einer erfindungsgemäßen Untersuchungseinrichtung in Form eines weiteren Typs einer Magnetresonanzeinrichtung.

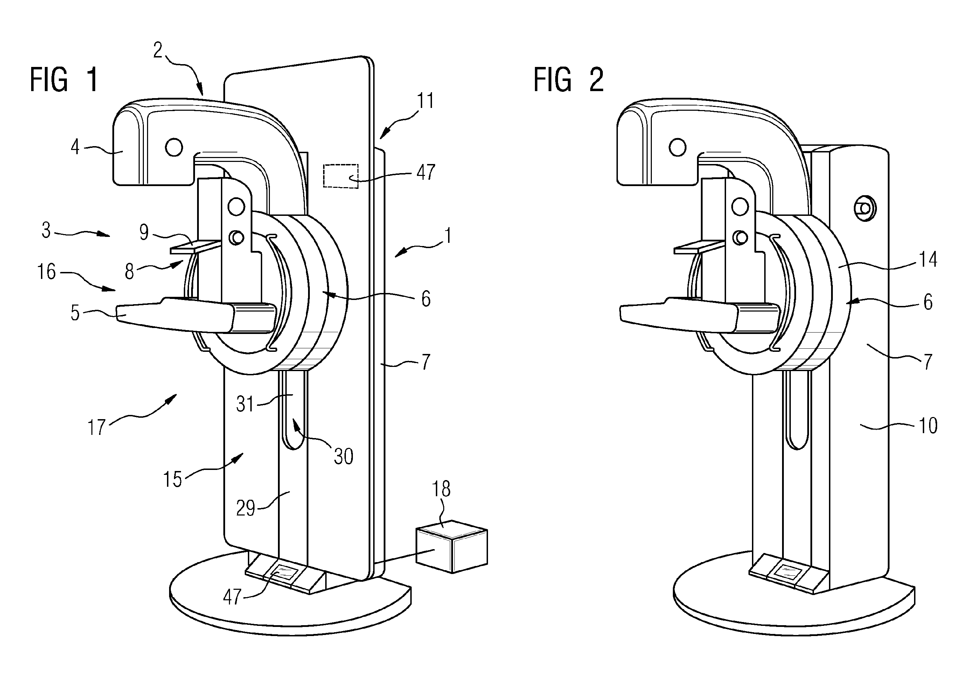

Fig. 1 zeigt eine erfindungsgemäße Untersuchungseinrichtung 1 in Form eines Röntgenmammographiegeräts 2 umfassend Röntgenbildaufnahmemittel 3 mit einer in einem oberen Arm 4 angeordneten, nicht näher gezeigten Röntgenstrahlenquelle und einem in einer unteren Auflage 5 angeordneten, nicht näher gezeigten Detektor. Die Röntgenbildaufnahmemittel 3 sind an einer Bewegungseinrichtung 6 angeordnet, die eine Vertikalbewegung längs einer Vertikalsäule 7 ermöglicht, um die Vertikalposition der Röntgenbildaufnahmemittel 3 der Größe der Patientin anzupassen. Mit dem Röntgenbildaufnahmemittel 3 vertikal bewegbar ist ferner eine Kompressionseinrichtung 8, über die für die Bildaufnahme die Brust mittels der vertikalbeweglichen Kompressionsplatte 9 komprimiert werden kann.

Die Bewegung erfolgt über eine geeignete Motorik, die im Bereich der Vertikalsäule 7 angeordnet ist, an welcher die Bewegungseinrichtung 6 vertikal bewegbar in an sich bekannter Weise geführt ist. Der grundsätzliche Aufbau eines solchen Mammographiegeräts 2 ist dem Grunde nach bekannt und muss nicht näher beschrieben werden.

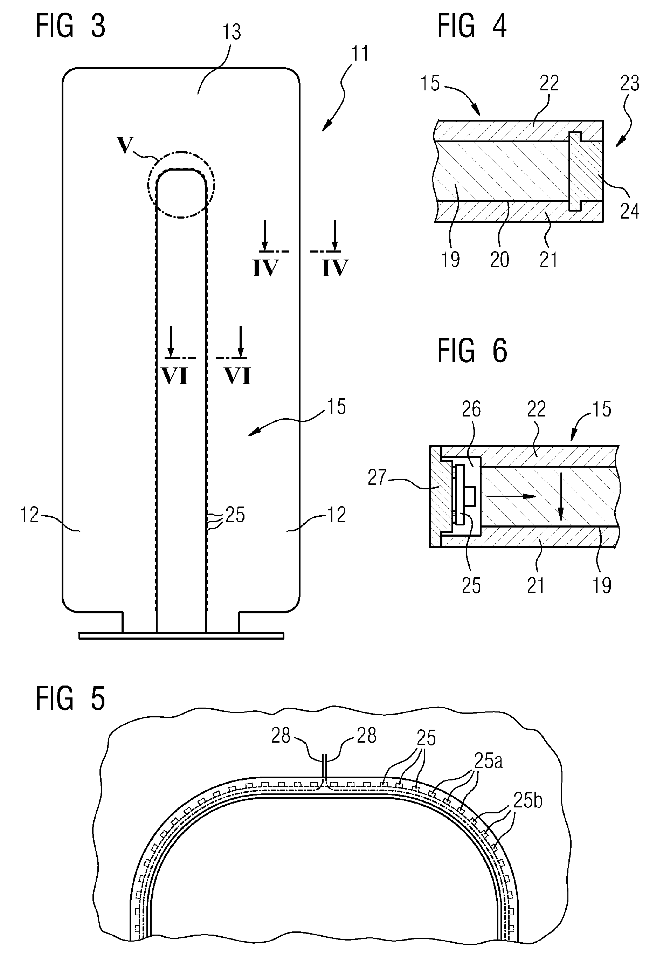

Zwischen die Vertikalsäule 7 bzw. deren äußeren Verkleidung 10 und das Röntgenbildaufnahmemittel 3 bzw. hier die im Wesentlichen kreisförmig eingehauste Bewegungseinrichtung 6 ist, siehe Fig. 1, eine flächige Leuchteinrichtung 11 integriert, die, siehe Fig. 3, im Wesentlichen eine U-Form mit zwei Seitenschenkeln 12 und einem Querschenkel 13 aufweist. Die beiden Seitenschenkel 12 werden von einem Tragarm, der Teil der Bewegungseinrichtung 6 ist, durchgriffen, über welchen Tragarm die Bewegungskopplung zur Verschiebemimik der Vertikalsäule 7 realisiert ist. Die Leuchteinrichtung 11 ist plattenförmig ausgeführt und sehr schmal, so dass sie ohne weiteres in den Zwischenraum zwischen der Vertikalsäulenverkleidung 10 und die Bewegungseinrichtung 6 bzw. deren Gehäuse 14 integriert werden kann. Zu diesem Zweck kann beispielsweise die Vertikalsäulenverkleidung 10 etwas zurückversetzt werden, um den Spalt zwischen ihr und der Verkleidung 14 der Bewegungseinrichtung 6 etwas zu vergrößern. Die Befestigung der plattenförmigen, großflächigen Leuchteinrichtung 11 erfolgt über geeignete Befestigungsmittel wie Verbindungsschrauben, Schnellbefestigungen unter Verwendung von Kniehebeln etc. an entsprechenden Befestigungsmitteln an der Vertikalsäulenverkleidung 10.

Die Leuchteinrichtung 11 weist eine Lichtabstrahlfläche 15 auf, über die Licht nach vorne in Richtung des Untersuchungsbereichs 16, der hier zwischen der Röntgenröhre und dem Strahlungsdetektor liegt, sowie des Bereichsumfelds 17, also den Bereich seitlich des Untersuchungsbereichs, emittiert werden kann. Die Lichtabstrahlfläche 15 entspricht im gezeigten Beispiel der gesamten Frontfläche der Leuchteinrichtung 11, über diese kann also großflächig und im Wesentlichen homogen Licht emittiert werden. Selbst wenn wie Fig. 1 zeigt über die Röntgenbildaufnahmemittel 3 bzw. die Bewegungseinrichtung 6 Teile der Lichtabstrahlfläche 15 verdeckt werden, liegen, resultierend aus der immensen Größe der Lichtabstrahlfläche 15, noch genügend große Flächenbereiche frei, um ausreichend viel Licht in den Untersuchungsbereich 16 und/oder das Bereichsumfeld 17 zu emittieren. Der Betrieb der Leuchteinrichtung 11 wird, wie in Fig. 1 nur exemplarisch auch für alle folgenden Ausführungsbeispiele geltend gezeigt ist, über eine entsprechende Steuerungseinrichtung 18 gesteuert, die die Vielzahl der Leuchtmittel ansteuert, worauf nachfolgend noch eingegangen wird. Bei dieser Steuerungseinrichtung 18 kann es sich selbstverständlich um die zentrale Steuerung des Mammographiegeräts 2 (im Falle des anderen Ausführungsbeispiels des jeweiligen Untersuchungsgeräts) handeln.

Die Figuren 3 - 6 zeigen in detaillierter Form den Aufbau der Leuchteinrichtung 11. Die Leuchteinrichtung 11 besteht aus drei Flächenbauteilen, nämlich einem ersten lichttransparenten und lichtleitenden Flächenelement 19, das beispielsweise aus Glas oder transparentem Kunststoff wie PMMA oder PC sein kann. Auf seine Rückseite 20 ist entweder eine Beschichtung aufgebracht, mittels der Lichtreflexions- bzw. Lichtstreuzentren realisiert werden, um von der Seite her eingekoppeltes Licht, worauf nachfolgend noch eingegangen wird, im Wesentlichen homogen zur Lichtabstrahlfläche 15 hin zu reflektieren. Rückseitig ist eine Rückwand 21 vorgesehen, die die Leuchteinrichtung 11 zur Rückseite hin, also zur Seite der Vertikalsäule 7, abschließt. An der gegenüber liegenden Seite befindet sich ein weiteres Flächenelement in Form eines Diffusors 22, bei dem es sich ebenfalls um eine beispielsweise sattinierte Glasscheibe oder eine entsprechend sattinierte Kunststoffscheibe handeln kann. Dieser Diffusor 22 bildet den vorderseitigen Abschluss und die frontseitige Lichtabstrahlfläche 15. Im Bereich des Außenrandes 23 ist ein Kantenabschlussbauteil 24 angeordnet, das im gezeigten Beispiel querschnittlich T-förmig ist und in entsprechende Nuten an der Rückwand 21 und dem Diffusor 22, die randkantenseitig ausgebildet sind, eingesetzt ist. Das Kantenabschlussbauteil 24 ist nicht transparent, es kann an seiner zur Innenseite hin gerichteten Seite sogar reflektierend ausgebildet sein (z. B. poliert oder mit einer Beschichtung belegt), um Licht wieder in das Flächenelement 19 zurückzureflektieren.

Das Licht, das über die Lichtabstrahlfläche 15 abgestrahlt wird, wird über eine Vielzahl einzelner Leuchtmittel 25 von der inneren Seitenkante der Leuchteinrichtung 11 bzw. des lichttransparenten lichtleitenden Flächenelements 19 her in dieses eingekoppelt. Wie Fig. 6 zeigt, sind im Bereich der Innenkanten die Rückwand 21 und der Diffusor 22 etwas weiter herausgezogen, so dass sich ein Aufnahmeraum 26 ausbildet, in dem die Leuchtmittel 25, hier eine Vielzahl von zu einer bandförmigen Einheit zusammengefassten LEDs, aufgenommen sind. Diese sind auf ein inneres Kantenabschlussbauteil 27 vorzugsweise aufgeklebt, das wiederum mit der Rückwand 21 und dem Diffusor 22 verklebt oder dort sonst wie befestigt ist. Wie durch den Pfeil in Fig. 6 gezeigt, wird das Licht quasi parallel zur Lichtabstrahlfläche 15 eingekoppelt, über die Vielzahl der an der Rückseite 20 ausgebildeten, gleichmäßig verteilten Reflexionszentren wird das Licht, wie durch den zweiten Pfeil in Fig. 6 gezeigt, zur Lichtabstrahlfläche 15 hin reflektiert, über die es dann austreten kann.

Aufgrund der lichtleitenden bzw. lichtführenden Eigenschaften des Flächenelements 19 und der Anordnung der Vielzahl der Leuchtmittel 25 über die gesamte Länge der Innenkante des Flächenelements 19 wird das Licht in dem gesamten Flächenelement 19 verteilt. Reflexionen in Richtung der Lichtabstrahlfläche 15 finden also über die gesamte Fläche der Rückseite 20 statt, wenn diese insgesamt mit Reflexionszentren in Form der Beschichtung oder durch unmittelbare Strukturierung der Rückseite 20 selbst versehen ist. Dies ermöglicht es, dass die Leuchteinrichtung über die gesamte Lichtabstrahlfläche 15 äußerst homogen, also in seiner Helligkeitsverteilung gleichmäßiges Licht emittiert und folglich der Untersuchungsbereich 16 wie auch der Umfeldbereich 17 mit homogenem Licht gleichförmig ausgeleuchtet werden können.

Wie Fig. 5 zeigt, sind die einzelnen Leuchtmittel 25 allesamt nebeneinander in einer Reihe angeordnet. Wie Fig. 5 ferner zeigt, werden im oberen Bereich der Ausnehmung zwischen den beiden Schenkeln 12 die Verbindungsleitungen 28 der hier gezeigten beiden Leuchtmittelstränge, die sich zur rechten und linken Seite erstrecken, herausgeführt und zur Steuerungseinrichtung 18 bzw. zu einer nicht näher gezeigten Stromquelle geführt. Bei den Leuchtmitteln 25, wie beschrieben bevorzugt LEDs, kann es sich insgesamt um weißes Licht emittierende Leuchtmittel, also LEDs, handeln. In diesem Fall wäre über die Leuchteinrichtung 11 nur weißes Licht abstrahlbar. Die emittierte Helligkeit kann über die Steuerungseinrichtung 18 eingestellt werden. Beispielsweise kann die Helligkeit zu Beginn einer Untersuchung, wenn die Patientin zu positionieren ist, oder wenn beispielsweise eine Biopsie zu entnehmen ist, auf maximal gestellt werden. Wenn dann die eigentliche Bildaufnahme beginnt, kann die Helligkeit etwas zurückgenommen werden, was dem Patientenkomfort zuträglich ist.

Alternativ zur Verwendung von nur weißes Licht emittierenden Leuchtmitteln 25 ist es auch denkbar, entweder abwechselnd oder nur in bestimmten Bereichen Leuchtmittel 25 vorzusehen, die farbiges Licht emittieren können. Beispielsweise kann jede zweite LED eine rotes Licht emittierende LED sein, oder in einem ausgezeichneten Längenabschnitt zu beiden Seiten der inneren Leuchteinrichtungskante können eine bestimmte Anzahl an rotes Licht emittierenden LEDs vorgesehen sein, wobei diese Farblicht emittierenden LEDs bzw. allgemein Leuchtmittel über die Steuerungseinrichtung 18 separat ansteuerbar sind. Dies ermöglicht es, im Bedarfsfall entweder vollständig oder bereichsweise, je nachdem, wie die Anordnung dieser Farblicht-LEDs ist, von einer weißen Beleuchtung auf eine farbige Beleuchtung umzuschalten, oder beide gemeinsam zu erzeugen. Dies bietet die Möglichkeit, über die Leuchtfarbe der Patientin Informationen zu geben, beispielsweise dahingehend, dass die Bildaufnahme begonnen hat, verbunden mit der daraus entnehmbaren Information, vollkommen ruhig zu stehen, wie auch bei Erlöschen der Farbbeleuchtung das Ende der Bildaufnahme angezeigt werden kann. Dabei ist es denkbar, auch die Farbbeleuchtung über die Steuerungseinrichtung 18 beliebig in ihrer Helligkeit variieren zu können, bzw. weißes und farbiges Licht in entsprechenden Helligkeitsstufen schalten zu können, bzw. beide gegensinnig auf- und abzudimmen, wenn ein vollständiger, vollflächiger Farbwechsel erfolgen soll.

Weiterhin ist es denkbar, anstelle von dezidierten Weißlicht- und Farblicht-Leuchtmitteln 25 solche zu verwenden, die unterschiedliches Farblicht emittieren, wobei durch entsprechende Lichtmischung an der Lichtabstrahlfläche 15 die gewünschte Lichtfarbe abgestrahlt wird. Beispielsweise können jeweils drei nebeneinander angeordnete Leuchtmittel 25, insbesondere in diesem Fall LEDs, zu jeweiligen Gruppen zusammengefasst werden und jeweils eine rotes Licht, eine grünes Licht und eine blaues Licht emittierende LED umfassen, wie durch die beiden Dreiergruppen 25a und 25b dargestellt ist. Jede einzelne LED innerhalb einer solchen Dreiergruppe ist separat über die Steuerungseinrichtung 18 ansteuerbar, wobei bevorzugt alle gleichfarbigen LEDs, die an der Seitenkante verteilt angeordnet sind, stets gemeinsam bzw. simultan angesteuert werden können. Auf diese Weise ist es durch geschickte Lichtmischung möglich, einerseits weißes Licht zu erzeugen, andererseits auch jede beliebige Mischfarbe. Dies bietet die Möglichkeit, über die gesamte Abstrahlfläche 15 sowohl hinsichtlich der Helligkeit bzw. Intensität als auch der Lichtfarbe homogenes Licht abstrahlen zu können. Beispielsweise kann zu Beginn der Untersuchung, wenn die Patientin an das Mammographiegerät 2 herantritt und zu positionieren ist, weißes Licht erzeugt und abgestrahlt werden. Nach erfolgter Positionierung kann dann auf eine beispielsweise von der Patientin gewünschte Lichtfarbe umgeschaltet werden, die dann über die Lichtabstrahlfläche 15 abgegeben wird. Die Steuerungseinrichtung 18 steuert dann die entsprechenden LEDs in der zur Erzeugung gewünschten Lichtfarbe erforderlichen Weise an. Hierüber kann eine angenehme Atmosphäre für die Patientin im Zusammenhang mit einer solchen häufig als unangenehm empfundenen Untersuchung erzeugt werden, wie hierüber natürlich auch entsprechende Informationen vermittelt werden können, selbst wenn zuvor die Lichtfarbe von Weißlicht auf eine gewünschte Lichtfarbe geändert wurde. Denn es ist ohne weiteres möglich, zu Beginn der eigentlichen Bildaufnahme, insbesondere dann, wenn nacheinander mehrere separate Bildaufnahmen aus verschiedenen Positionen erfolgen sollen, beispielsweise auf Rotlicht umzuschalten, um der Patientin die Bildaufnahme zu signalisieren, und am Ende der Bildaufnahme wieder auf die gewünschte Lichtfarbe umzuschalten. Denkbar sind hier beliebige Farbvariationen und Steuerungsabfolgen.

Alternativ zur gezeigten Anordnung der Leuchtmittel 25 an der inneren Seitenkante der Leuchteinrichtung 11 besteht selbstverständlich die Möglichkeit, diese außenseitig anzuordnen und innenseitig einen lichtdichten, gegebenenfalls an der Innenseite selbst reflektierenden Abschluss in Form eines Kantenabschlussbauteils anzuordnen. Auch ist die Form der Leuchteinrichtung selbstverständlich nicht auf die gezeigte Rechteckform beschränkt. Selbstverständlich wäre es denkbar, die Ausgestaltung auch oval auszuführen, um in dem Bereich, in dem die Bewegungseinrichtung 6 vor der Lichtabstrahlfläche 15 verfahren wird, zur Seite hin einen größeren Abstrahlflächenbereich zu realisieren.

Wie Fig. 1 ferner zeigt, ist zwischen den beiden Schenkeln 12 der Leuchteinrichtung 11 ein Abdeckelement 29 vorgesehen, das eine mittige Ausspaltung 30 aufweist, in der eine Jalousie 31 angeordnet ist, die den dahinter befindlichen Raum zur Vertikalsäule hin schließt. Das Abdeckelement 29 hat weiterhin den Zweck, an der Vorderseite, also der Lichtabstrahlfläche 15, die Leuchtmittel 25 abzudecken, so dass es nicht möglich ist, von vorne oder der Seite direkt auf diese zu blicken. Das heißt, der Randbereich des Abdeckelements 29 greift etwas über die Lichtabstrahlfläche 15 im Randkantenbereich, in dem die Leuchtmittelstränge verlaufen.

Vorgesehen ist des Weiteren ein Display 47 am Fuß der Untersuchungseinrichtung, an dem für den behandelnden Arzt etc. Informationen dargestellt werden können. Ein solches Display 47, z.B. ein sehr flaches LCD-Display, kann zusätzlich oder alternativ auch an der Leuchteinrichtung 11 vorgesehen sein, wie in Fig. 2 gestrichelt dargestellt ist. Hierzu ist z.B. der Diffusor 22 mit einer Ausnehmung versehen, in die das Display eingesetzt ist, etwaige Leitungsverbindungen können über eine Durchbrechung im Flächenelement 19 und der Rückwand 21 nach hinten geführt werden. Denkbar wäre es auch, das sehr flache Display direkt auf den Diffusor 22 aufzusetzen, so dass die Leuchteinrichtung in ihrem Aufbau grundsätzlich unverändert bleibt. An dem Display können bei Anordnung in dieser Position auch für die Patientin geltende Informationen dargestellt werden. Die Integration eines solchen Displays ist, wenngleich nicht näher beschrieben, bei allen Leuchteinrichtungen der nachfolgend noch beschriebenen Ausführungsbeispiele möglich.

Anstelle eines Displays kann dieser Bereich auch als Projektionsfläche für eine z.B. am Arm 4 befindliche Projektionseinrichtung, z.B. einen Beamer, dienen, mit dem Informationen auf die Projektionsfläche gestrahlt werden können. Dies ist u.U. auch bei betriebener Leuchteinrichtung 11 möglich, wenn die Lichtleistung der Projektionseinrichtung groß genug ist. Auch der Einsatz einer Projektionseinrichtung und einer Projektionsfläche ist bei den nachfolgend noch beschriebenen Ausführungsformen möglich.

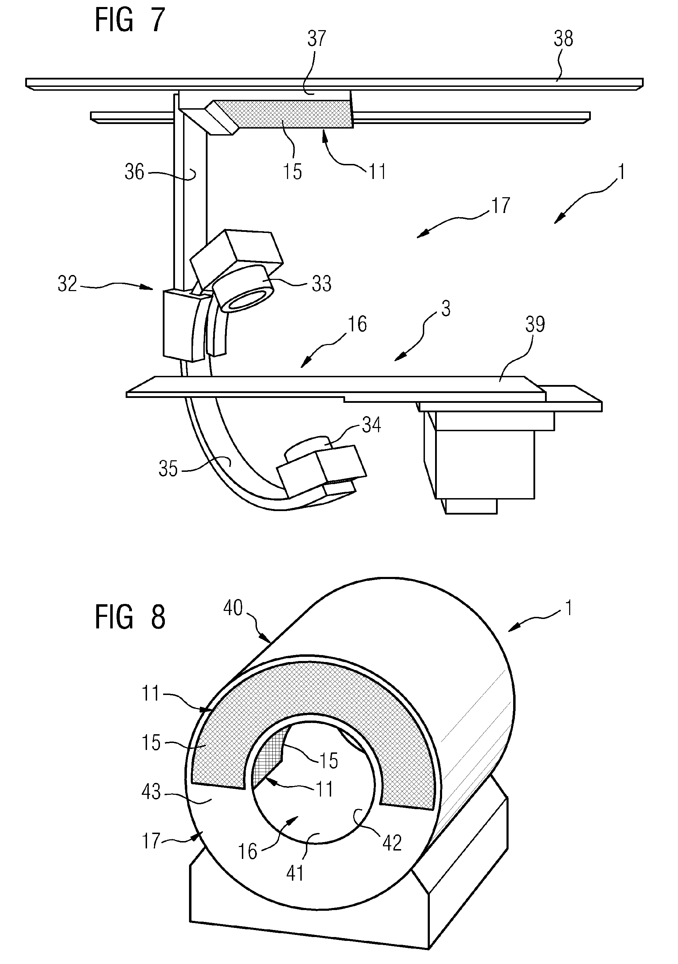

In den Figuren 7 - 9 werden nachfolgend weitere Beispiele erfindungsgemäßer Untersuchungseinrichtungen unterschiedlicher Gattung beschrieben, die jeweils wenigstens eine Leuchteinrichtung 11 aufweisen, die vom grundsätzlichen Aufbau der Leuchteinrichtung 11, wie sie zur Ausgestaltung gemäß der Figuren 1 - 6 beschrieben wurde, aufgebaut ist, so dass diesbezüglich und bezüglich des Funktionsprinzips etc. zu den einzelnen nachfolgend beschriebenen Ausführungsformen nichts näher beschrieben wird. Soweit möglich, werden für gleiche Elemente die gleichen Bezugszeichen verwendet.

Fig. 7 zeigt eine weitere erfindungsgemäße Untersuchungseinrichtung 1 in Form eines C-Bogen-Röntgengeräts 32. Dieses umfasst ein Bildaufnahmemittel 3 mit einer Röntgenröhre 33 und einem Detektor 34, die beide an einem C-Bogen 35 angeordnet sind, der wiederum an einem Stativ 36 befestigt ist, das seinerseits an einem Wagen 37, der auf geeigneten deckenseitig angeordneten Führungsmitteln 38 in x- und gegebenenfalls y-Richtung verfahrbar ist, angeordnet ist. Vorgesehen ist ferner ein Patientenlagerungstisch 39, auf den ein Patient aufgebracht wird, der zu untersuchen ist. Der Untersuchungsbereich 16 befindet sich zwischen Röntgenröhre 33 und Detektor 34, das Bereichsumfeld 17 primär oberhalb des Patientenlagerungstischs 39, wo sich also der Patient befindet.

Auch hier ist erfindungsgemäß eine Leuchteinrichtung 11 vorgesehen, die hier an der Unterseite des Wagens 37 ebenfalls als großflächige Leuchteinrichtung angeordnet ist. Sie weist hier eine Rechteckform auf, entsprechend der Grundform des Wagens 37, kann aber jede beliebige andere Form aufweisen und sogar über die Wagengrundform seitlich überstehen. Das Licht über die Lichtabstrahlfläche 15 wird hier nach unten reflektiert, nachdem sich der Untersuchungsbereich 16 und das Bereichsumfeld 17 darunter befinden. Das emittierte Licht ist auch hier infolge des Aufbaus der Leuchteinrichtung 11 über die gesamte Lichtabstrahlfläche 15 äußerst homogen, wie auch bei Verwendung entsprechender Leuchtmittel 25 sowohl weißes als auch farbiges Licht abgestrahlt werden kann. In diesem Zusammenhang sei auf die Ausführungen zuvor verwiesen.

Auch bei einer solchen Untersuchungseinrichtung 1 besteht folglich die Möglichkeit, den Untersuchungsbereich 16 wie auch das Bereichsumfeld 17 beispielsweise zu Beginn der Untersuchung, wenn der Patient auf dem Patientenlagerungstisch 39 aufzubringen ist und mit diesem bezüglich der Bildaufnahmeeinrichtung 3 zu positionieren ist, mit Weißlicht sehr hell auszuleuchten, so dass die die Untersuchung durchführende Person ein optimal und hell ausgeleuchtetes Arbeitsumfeld hat. Zu Beginn der Röntgenbildaufnahme kann dann die Helligkeit zurückgenommen oder auf Farblicht etc. umgeschaltet werden, sofern dies erforderlich ist, wobei auch hier eine Informationsvermittlung über die Lichtfarbe erreicht werden kann. Der Patient, der häufig mit dem Gesicht zur Leuchteinrichtung 11 blickend angeordnet ist, kann einen etwaigen Farbwechsel sofort erkennen, so dass ihm hierüber beispielsweise angezeigt werden kann, dass er sich ab dem Farbwechsel nicht mehr bewegen darf, etc. Auch kann über die entsprechende Lichtfarbe, sofern gewünscht und erforderlich, eine entsprechend den Patientenkomfort fördernde Beleuchtungsatmosphäre geschaffen werden, insbesondere bei lang dauernden Bildaufnahmen.

Fig. 8 zeigt eine weitere Ausgestaltung einer erfindungsgemäßen Untersuchungseinrichtung 1 in Form eines Magnetresonanzgeräts 40, alternativ kann es sich hierbei auch um einen Computertomographen handeln. Unabhängig vom nun konkreten Gerätetyp weist diese Untersuchungseinrichtung eine hier zylindrische Aufnahmebohrung 41 auf, in die der Patient mittels eines hier nicht näher gezeigten Patientenlagerungstisches, der zur Patientenaufnahme außerhalb der Aufnahmebohrung 41 positioniert ist, eingefahren wird. Der Untersuchungsbereich 16 befindet sich hier im Inneren der Aufnahmebohrung 41, primär dort in der Mitte, das Bereichsumfeld 17 befindet sich primär außerhalb der Aufnahmebohrung 41, kann sich aber auch noch etwas in diese erstrecken.

In jedem Fall ist im Inneren der Aufnahmebohrung 41 am vorderen Bohrungsrand erfindungsgemäß eine Leuchteinrichtung 11 angeordnet, die sich eine gewisse Länge in die Aufnahmebohrung 41 hineinerstreckt, jedoch lediglich so weit, dass die Bildaufnahme, die im Falle einer Magnetresonanzeinrichtung unter Verwendung geeigneter Magnetfelder erfolgt, hierüber nicht gestört wird. Die Leuchteinrichtung 11 ist beispielsweise auf die Innenverkleidung 42 der Bohrung im oberen Bereich aufgesetzt oder ersetzt diese lokal. Sie ist infolge der Zylinderform der Aufnahmebohrung 41 gebogen und erstreckt sich im gezeigten Beispiel um etwas mehr als 180°. Die Lichtabstrahlfläche 15 ist hier nach unten gerichtet, also zum Patienten hin, der unter der Leuchteinrichtung 11 eingefahren wird bzw. dort oder in deren Beleuchtungsbereich positioniert ist.

Mittels dieser Leuchteinrichtung 11 ist es also möglich, einen Teil des Untersuchungsbereichs 16 bzw. des davor befindlichen Bereichsumfelds 17 homogen auszuleuchten. Der Aufbau der hier gebogenen Leuchteinrichtung 11 entspricht dem einleitend beschriebenen Aufbau. Die Leuchtmittel sind bevorzugt an der vorderen oder hinteren, langen Stirnkante angeordnet.

Nachdem sich häufig der Kopf des Patienten, wenn dieser nicht gerade zu untersuchen ist, im Bereich des Eingangs der Patientenaufnahme befindet, kann ihm hierüber ohne weiteres eine lichtoptische Information gegeben werden, wie bereits mehrfach beschrieben wurde. Auch kann hierüber vor und während der Untersuchung eine optimale Bereichsausleuchtung auch für die die Untersuchung durchführende Person erreicht werden, wie auch eine angenehme Beleuchtungsatmosphäre insbesondere bei lang dauernden Untersuchungen geschaffen werden kann.

All dies ist bei der Untersuchungseinrichtung 1 aus Fig. 8 ferner über eine zweite Leuchteinrichtung 11 möglich, die an der Stirnseite 43 der Magnetresonanzeinrichtung 40 oder andernfalls des Computertomographen vorgesehen ist. Die zusätzliche oder alternative Leuchteinrichtung 11 ist hier U-förmig ausgeführt und erstreckt sich im gezeigten Beispiel um ca. 180° um die Aufnahmebohrung 41 herum. Auch sie bietet die Möglichkeit einer großflächigen Lichtabstrahlung über die Lichtabstrahlfläche 15. Die Leuchtmittel können auch hier, ähnlich wie bei der Leuchteinrichtung 11 aus Fig. 1, im Bereich der inneren Kante sein, eine außenseitige Anordnung ist jedoch gleichermaßen denkbar. Auch kann es sich um beliebige Leuchtmittel, die weißes oder farbiges Licht emittieren, handeln. Wiederum sei auf die vorherigen Ausführungen verwiesen. Diese zweite Leuchteinrichtung 11 strahlt nach vorne in den Umfeldbereich 17 vor der Untersuchungseinrichtung 1 ab, wo sich also zur Patientenaufnahme der ausgefahrene Patientenlagerungstisch etc. befindet. Dies bietet die Möglichkeit, diesen Bereich optimal ausleuchten zu können, um entsprechende vorbereitende Handlungen am Patienten wie beispielsweise das Anordnen von Lokalspulen etc. vornehmen zu können.

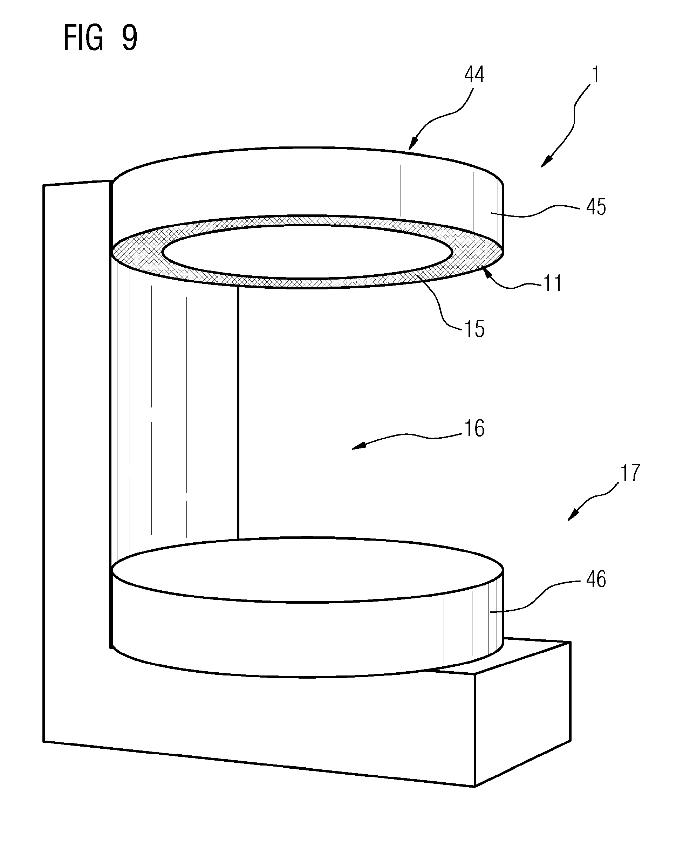

Fig. 9 zeigt schließlich eine weitere erfindungsgemäße Untersuchungseinrichtung 1 in Form einer Magnetresonanzeinrichtung 44, die zwei separate, horizontal übereinander angeordnete Magneten 45, 46 aufweist, zwischen denen sich der Untersuchungsbereich 16 und außerhalb derer sich das Bereichsumfeld 17 befindet. Am oberen Magneten 45 ist eine Leuchteinrichtung 11 angeordnet, die hier ringförmig ist und um den Rand des Magneten 45 umläuft. Die Lichtabstrahlfläche ist auch hier nach unten zum Untersuchungsbereich 16 gerichtet, so dass auch dieser optimal und homogen ausgeleuchtet werden kann. Der Aufbau der Leuchteinrichtung 11 entspricht dem beschriebenen Aufbau, auch hier können die Leuchtmittel bevorzugt an der inneren Seitenkante angeordnet sein. Wiederum kann es sich um weißes Licht oder farbiges Licht emittierende LEDs handeln. Die erreichbaren Vorteile entsprechen den zuvor beschriebenen Vorteilen, wie sie mit allen erfindungsgemäßen Untersuchungseinrichtungen 1 erreicht werden können.

Die Befestigung einer jeden Leuchteinrichtung 11, egal woran sie nun angeordnet ist, erfolgt über geeignete Verbindungsmittel wie Schrauben, Schnellverbinder in Form von Kniehebeln, Bajonettverschlüssen etc. Hierzu können an der Rückwand 21 jeder Leuchteinrichtung 11 entsprechende Befestigungsmittel in Form von Halteschienen oder Haltewinkeln etc. angeordnet sein, die mit entsprechenden Befestigungsmitteln am jeweiligen Bauteil der Untersuchungseinrichtung zu verbinden sind. Bei einem solchen Bauteil kann es sich beispielsweise um ein Verkleidungsteil handeln, auf das die Leuchteinrichtung 11 aufgesetzt wird, oder eine Rahmenkonstruktion beispielsweise des Wagens 37 etc. Die Art und Positionierung der leuchteinrichtungsseitigen Befestigungsmittel richtet sich selbstverständlich nach der Befestigungsmöglichkeit am entsprechenden Bauteil der Untersuchungseinrichtung.

### Bezugszeichenliste

- 1: Untersuchungseinrichtung
- 2: Röntgenmammographiegerät
- 3: Röntgenbildaufnahmemittel
- 4: Oberer Arm
- 5: Untere Auflage
- 6: Bewegungseinrichtung
- 7: Vertikalsäule
- 8: Kompressionseinrichtung
- 9: Kompressionsplatte
- 10: Vertikalsäulenverkleidung
- 11: Leuchteinrichtung
- 12: Seitenschenkel
- 13: Querschenkel
- 14: Verkleidung
- 15: Lichtabstrahlfläche
- 16: Untersuchungsbereich
- 17: Bereichsumfeld
- 18: Steuerungseinrichtung
- 19: Flächenelement
- 20: Rückseite
- 21: Rückwand
- 22: Diffusor
- 23: Außenrand
- 24: Kantenabschlussbauteil
- 25: Leuchtmittel
- 26: Aufnahmeraum
- 27: Kantenabschlussbauteil
- 28: Verbindungsleitungen
- 29: Abdeckelement
- 30: Ausspaltung
- 31: Jalousie
- 32: C-Bogen-Röntgengerät
- 33: Röntgenröhre
- 34: Detektor
- 35: C-Bogen
- 36: Stativ
- 37: Wagen
- 38: Führungsmittel
- 39: Patientenlagerungstisch
- 40: Magnetresonanzgerät
- 41: Aufnahmebohrung
- 42: Innenverkleidung
- 43: Stirnseite
- 44: Magnetresonanzeinrichtung
- 45: Magnet
- 46: Magnet

## Patentansprüche

1. Medizinische Untersuchungsvorrichtung umfassend einen Untersuchungsbereich, in dem eine Untersuchungsperson unter Verwendung einer bildgebenden Einrichtung untersucht wird, **dadurch gekennzeichnet, dass** wenigstens eine flächige, über ihre Lichtabstrahlfläche (15) homogenes Licht emittierende Leuchteinrichtung (11) umfassend wenigstens ein Leuchtmittel (25, 25a, 25b) vorgesehen ist, mittels der zumindest ein Teil des Untersuchungsbereichs (16) und/oder des Bereichsumfelds (17) ausleuchtbar ist.

2. Untersuchungseinrichtung nach Anspruch 1, d a - **durch gekennzeichnet, dass** die Leuchteinrichtung (11) im wesentlichen plattenförmig und eben oder gebogen ausgebildet ist und den Untersuchungsbereich (16) oder das Bereichsumfeld (17) zumindest teilweise begrenzt

3. Untersuchungseinrichtung nach Anspruch 1 oder 2, **dadurch gekennzeichnet, dass** die Leuchteinrichtung (11) ein lichtleitendes Flächenelement (19) umfasst, das ein Mittel zur Umlenkung von seitlich über das wenigstens eine Leuchtmittel (25, 25a, 25b) eingekoppelten Lichts zur Lichtabstrahlfläche (15) aufweist.

4. Untersuchungseinrichtung nach Anspruch 3, d a - **durch gekennzeichnet, dass** das Mittel zur Umlenkung eine auf eine Seite des Flächenelements (19) aufgebrachte Beschichtung oder eine Strukturierung der Oberfläche des Flächenelements (19) ist.

5. Untersuchungseinrichtung nach Anspruch 3 oder 4, **dadurch gekennzeichnet, dass** das Flächenelement (19) einen an der Seite der Lichtabstrahlfläche (15) vorgesehenen Diffusor (22) aufweist oder dieser Seite ein Diffusor (22) zugeordnet ist.

6. Untersuchungseinrichtung nach einem der Ansprüche 3 bis 5, **dadurch gekennzeichnet, dass** entlang der Seite mehrere Leuchtmittel (25, 25a, 25b) zum Einkoppeln von Licht in das Flächenelement (19) vorgesehen sind.

7. Untersuchungseinrichtung nach Anspruch 6, d a - **durch gekennzeichnet, dass** die mehreren Leuchtmittel (25, 25a, 25b) in einer bandartigen Einheit zusammengefasst sind.

8. Untersuchungseinrichtung nach einem der vorangehenden Ansprüche, **dadurch gekennzeichnet, dass** das oder die Leuchtmittel (25, 25a, 25b) LEDs sind.

9. Untersuchungseinrichtung nach einem der vorangehenden Ansprüche, **dadurch gekennzeichnet, dass** der Leuchteinrichtung (11) im Bereich der Lichtabstrahlfläche (15) ein das oder die Leuchtmittel (25, 25a, 25b) verdeckendes nicht transparentes Abdeckelement (29) zugeordnet ist.

10. Untersuchungsvorrichtung nach einem der vorangehenden Ansprüche, **dadurch gekennzeichnet, dass** die Leuchteinrichtung (11) eine Steuerungseinrichtung (18) umfasst, über welche die Helligkeit des oder der Leuchtmittel (25, 25a, 25b) veränderbar ist.

11. Untersuchungseinrichtung nach Anspruch 10, d a - **durch gekennzeichnet, dass** die Helligkeit über die Steuerungseinrichtung (18) zwischen diskreten Helligkeitsstufen schaltbar ist, oder dass die Helligkeit stufenlos zwischen einem Maximalwert und einem Minimalwert dimmbar ist.

12. Untersuchungseinrichtung nach einem der vorangehenden Ansprüche, **dadurch gekennzeichnet, dass** mittels der Leuchteinrichtung (11) weißes Licht und Licht wenigstens einer Farbe emittierbar ist.

13. Untersuchungseinrichtung nach Anspruch 12, **dadurch gekennzeichnet, dass** weißes und farbiges Licht in voneinander getrennten Bereichen der Lichtabstrahlfläche (15) abstrahlbar ist, oder dass über jeweils die gesamte Lichtabstrahlfläche (15) weißes oder farbiges Licht abstrahlbar ist.

14. Untersuchungseinrichtung nach Anspruch 12 oder 13, **dadurch gekennzeichnet, dass** die mehrere Leuchtmittel (25) umfassende Leuchteinrichtung (11) neben wenigstens einem weißes Licht emittierenden Leuchtmittel (25) wenigstens ein farbiges Licht emittierendes Leuchtmittel (25) umfasst, das separat ansteuerbar ist.

15. Untersuchungseinrichtung nach Anspruch 12 oder 13, **dadurch gekennzeichnet, dass** wenigstens ein rotes Licht, wenigstens ein blaues Licht und wenigstens ein grünes Licht emittierendes Leuchtmittel (25a, 25b) vorgesehen ist, die separat ansteuerbar sind.

16. Untersuchungseinrichtung nach Anspruch 15, d a - **durch gekennzeichnet, dass** die abgestrahlte Lichtfarbe stufenlos veränderbar ist.

17. Untersuchungseinrichtung nach einem der vorangehenden Ansprüche, **dadurch gekennzeichnet, dass** an oder in der Leuchteinrichtung (11) wenigstens ein der Darstellung von Informationen und/oder Bildern dienendes Display angeordnet ist, oder dass der Leuchteinrichtung (11) ein solches Display zugeordnet ist.

18. Untersuchungseinrichtung nach einem der vorangehenden Ansprüche, **dadurch gekennzeichnet, dass** wenigstens eine Projektionseinrichtung vorgesehen ist, mittels der Informationen und/oder Bilder auf einen Bereich der Lichtabstrahlfläche (15) der Leuchteinrichtung (11) projizierbar sind.

19. Untersuchungseinrichtung nach einem der vorangehenden Ansprüche, **dadurch gekennzeichnet, dass** es eine Röntgeneinrichtung ist.

20. Untersuchungseinrichtung nach Anspruch 19, **dadurch gekennzeichnet, dass** es eine der Untersuchung einer stehenden Untersuchungsperson dienende Untersuchungseinrichtung ist, wobei die Leuchteinrichtung (11) vertikal stehend angeordnet ist.

21. Untersuchungseinrichtung nach Anspruch 20, **dadurch gekennzeichnet, dass** es ein Mammografiegerät (2) ist, wobei die Leuchteinrichtung (11) hinter dem an einer Vertikalsäule (7) vertikal bewegbar angeordneten Röntgenbildaufnahmemittel (3) angeordnet ist.

22. Untersuchungseinrichtung nach Anspruch 19, **dadurch gekennzeichnet, dass** es ein Röntgengerät mit vertikal angeordnetem und gegebenenfalls an einer Vertikalsäule (7) vertikal beweglichem Detektor ist, wobei die Leuchteinrichtung (11) hinter dem Detektor angeordnet ist.

23. Untersuchungseinrichtung nach Anspruch 21 oder 22, **dadurch gekennzeichnet, dass** die Leuchteinrichtung (11) eine U-Form aufweist, und zwischen der Vertikalsäule (7) und den Röntgenbildaufnahmemitteln (3) oder dem Detektor angeordnet ist, wobei ein das Röntgenbildaufnahmemittel (3) oder den Detektor tragender und an der Vertikalsäule (7) geführter Tragarm zwischen den beiden U-Schenkeln (12) durchgreift.

24. Untersuchungseinrichtung nach Anspruch 19, d a - **durch gekennzeichnet, dass** das Röntgenbildaufnahmemittel (3) an einem Stativ (36), das an einem an deckenseitig angeordneten Führungsmitteln (38) bewegbar geführten Wagen (37) angeordnet ist, angeordnet ist, und ein Lagerungstisch (39) für die Untersuchungsperson vorgesehen ist, wobei die Leuchteinrichtung (11) an dem Wagen (37) angeordnet ist.

25. Untersuchungseinrichtung nach einem der Ansprüche 1 bis 18, **dadurch gekennzeichnet, dass** es eine Magnetresonanzeinrichtung (40) oder eine Computertomographieeinrichtung ist.

26. Untersuchungseinrichtung nach Anspruch 25, **dadurch gekennzeichnet, dass** sie eine im wesentlichen zylindrische Aufnahmebohrung (41) für die Untersuchungsperson aufweist, wobei die Leuchteinrichtung (11) diese zumindest über einen Teil ihrer Länge und zumindest über einen Teil ihres Umfangs auskleidet.

27. Untersuchungseinrichtung nach Anspruch 25 oder 26, **dadurch gekennzeichnet, dass** sie eine im wesentlichen zylindrische Aufnahmebohrung (41) für das Untersuchungsobjekt aufweist, wobei die Leuchteinrichtung (11) zumindest einen Teil der im wesentlichen ringförmigen Stirnseite (43) der Untersuchungseinrichtung (1) benachbart zur Aufnahmebohrung (41) belegt.

28. Untersuchungseinrichtung nach Anspruch 25, d a - **durch gekennzeichnet, dass** die Magnetresonanzeinrichtung (44) zwei horizontal angeordnete und voneinander beabstandete Magneten (45, 46) aufweist, zwischen denen sich der Untersuchungsbereich (16) befindet, wobei die Leuchteinrichtung (11) am oberen Magneten (45), diesen zumindest teilweise belegend, angeordnet ist.
